# FASCICULE DE BREVET EUROPEEN

(11) **EP 1 205 466 B1**
(45) Date de publication et mention de la délivrance du brevet: **31.03.2004**
(21) Numéro de dépôt: 01402756.9
(22) Date de dépôt: 24.10.2001
(51) Int. Cl.: C07C 66/02

(54) **Procédé de synthèse de la carboxy-2-anthraquinone par oxydation nitrique d'ethyl-2-anthraquinone**
Verfahren zur Herstellung von Anthrachinon-2-Carbonsäure durch Oxidation von Ethyl-2-anthrachinon mit Salpetersäure
Process for the synthesis of carboxy-2-anthraquinone by nitric oxidation of ethyl-2-anthraquinone

(30) Priorité: 13.11.2000 FR 0014545
(43) Date de publication de la demande: 15.05.2002
(73) Titulaire: Atofina, 92091 Paris La Défense (FR)
(72) Inventeur: Devic, Michel, 69110 Sainte Foy Les Lyon (FR)

(56) Documents cités:
- GB-A- 662 139
- GB-A- 1 425 692
- US-A- 3 165 548
- CHEMICAL ABSTRACTS, vol. 77, no. 17, 23 octobre 1972 (1972-10-23) Columbus, Ohio, US; abstract no. 114111g, K.I.PORSHAKOVA ET AL.: "Alkylation of anthracene with alcohols in the presence of zinc chloride" page 424; XP002175577 & TR.TASHKENT POLITEKH. INST., vol. 64, 1970, pages 55-61,
- DATABASE WPI Section Ch, Week 197531 Derwent Publications Ltd., London, GB; Class E14, AN 1975-51619W XP002175578 & JP 50 047964 A (KAWASAKI CHEM IND CHEM), 28 avril 1975 (1975-04-28)

## Description

La présente invention concerne un procédé de synthèse de la carboxy-2-anthraquinone à partir d'éthyl-2-anthraquinone par oxydation dudit groupement éthyl au moyen d'une solution d'acide nitrique.

Les anthraquinones sont utilisées dans la fabrication de films absorbeurs d'oxygène permettant l'élaboration d'emballages adaptés à la conservation de produits sensibles à l'oxygène tels que les produits alimentaires.

En effet, de nombreux produits alimentaires se dégradent au contact de l'oxygène. On utilise, en général, pour leur conservation un emballage comprenant une structure multicouche. Cette structure multicouche comprend, entre autre, un film barrière à l'oxygène et un film absorbeur d'oxygène. Ces films sont disposés de telle sorte que le film barrière à l'oxygène est en contact avec l'air externe à l'emballage scellé et le film absorbeur d'oxygène est disposé en contact avec l'aliment se trouvant à l'intérieur de l'emballage scellé.

Si le film barrière a pour but d'assurer une protection contre l'entrée d'oxygène à l'intérieur de l'emballage scellé, le film absorbeur d'oxygène assure l'absorption de l'oxygène resté éventuellement prisonnier à l'intérieur de l'emballage au moment de la fermeture de celui-ci ou émanant des aliments eux-mêmes.

La structure multicouche comprend également d'autres films, par exemple, en polyéthylène ou en polypropylène qui assurent la tenue mécanique de la structure et la protection contre l'humidité.

Plusieurs procédés de préparation de la carboxy-2-anthraquinone sont déjà décrits par oxydation de dérivés de l'anthraquinone ou de l'anthracène. Le brevet JP 50047964 décrit la préparation de carboxy-2-anthraquinone par oxydation de méthyl-2-anthraquinone par l'oxygène en présence de cobalt et de manganèse dans l'acide acétique.

K.I.Porshakova et al. décrivent dans "Alkylation of anthracene with alcohols in the presence of zinc chloride", TR.TASHKENT POLITEKH. INST., vol. 64, 1970, pages 55-61 (CHEMICAL ABSTRACTS, vol. 77, no. 17, 23 octobre 1972 (1972-10-23) Columbus, Ohio, US; abstract no. 114111 g, page 424, XP002175577) la préparation de carboxy-2-anthraquinone par oxydation de l'amyl-2-anthraquinone au moyen de l'acide nitrique.

Toutefois, les procédés connus de préparation de la carboxy-2-anthraquinone font appel à des oxydants très toxiques pour l'environnement comme par exemple, le chrome, le manganèse et le cobalt ou bien à des techniques ou à des réactifs difficiles à mettre en oeuvre comme par exemples, les rayonnements UV ou le chlore.

De plus, ces procédés de fabrication de la carboxy-2-anthraquinone ont pour inconvénient d'utiliser des matières premières onéreuses et principalement disponibles en petites quantités telles que la méthyl-2-anthraquinone ou les dérivés de l'anthracène.

L'invention vise donc à fournir une solution à ces inconvénients.

L'invention est relative à un procédé de synthèse de carboxy-2-anthraquinone à partir d'éthyl-2-anthraquinone par oxydation nitrique.

Selon un mode de réalisation de l'invention, l'oxydation nitrique est réalisée à l'aide d'une solution aqueuse d'acide nitrique à une concentration comprise entre 1 et 20 % en poids et de préférence entre 3 et 15 % en poids.

Selon un mode de réalisation de l'invention, la proportion d'éthyl-2-anthraquinone à traiter est comprise entre 2 et 20 % en poids de solution d'acide nitrique et de préférence entre 5 et 12 % en poids.

Selon un mode de réalisation de l'invention, la température de la réaction est comprise entre 120 et 220°C et de préférence entre 160 et 200°C.

Selon un mode de réalisation de l'invention, la pression à laquelle est réalisée la réaction est comprise entre 6 et 80 bars et de préférence entre 15 et 20 bars.

Selon un mode de réalisation de l'invention, la pression est maintenue constante tout au long de la réaction.

Selon un mode de réalisation de l'invention, les vapeurs d'oxydes d'azote produites au cours de la réaction sont recyclées en acide nitrique.

Nous allons maintenant décrire le procédé de fabrication de la carboxy-2-anthraquinone.

Le principe de la réaction consiste à mettre en présence de l'éthyl-2-anthraquinone avec une solution aqueuse d'acide nitrique à chaud et sous une certaine pression. Les produits de la réaction sont la carboxy-2-anthraquinone, du dioxyde de carbone et des oxydes d'azote.

S'agissant de la température de la réaction, celle-ci est comprise entre 120 et 220°C. Elle est de préférence comprise entre 160 et 200°C.

S'agissant de la pression, elle est supérieure ou égale à la pression de vapeur saturante de la solution d'acide nitrique à la température de la réaction. Si aucun dégazage n'est réalisé, la pression est supérieure à la pression saturante car la réaction génère des composés gazeux qui sont le dioxyde de carbone et des oxydes d'azote. La réaction est réalisée à une pression pouvant être comprise entre 6 et 80 bars. La pression est avantageusement comprise entre 15 et 20 bars, moyennant un ou des dégazages manuels ou automatiques au cours de la réaction, pour une température de réaction comprise entre 180 et 200°C.

S'agissant de la concentration de la solution aqueuse d'acide nitrique, elle est de 1 à 20 % en poids et, de préférence, elle est comprise entre 3 et 15 % en poids.

S'agissant de la quantité d'éthyl-2-anthraquinone introduite dans le milieu réactionnel, elle est comprise entre 2 et 20 % en poids de solution d'acide nitrique et de préférence entre 5 et 12 % en poids.

La réaction peut être effectuée :
- par un mode discontinu, c'est à dire par chargement des réactifs et déchargement des produits de façon discontinue ;
- par un mode semi-continu, c'est à dire par chargement continu des réactifs et déchargement discontinu des produits ; ou encore
- par un mode continu, c'est à dire par chargement des réactifs et déchargements des produits de façon continue.

De préférence, on utilise un mode discontinu pour les petites quantités de réactifs et produits. Pour les réalisations industrielles ou semi-industrielles, les modes semi-continu ou continu sont préférés, le mode semi-continu ayant l'avantage de permettre un bon contrôle de l'exothermie de la réaction et de la concentration en acide nitrique et un déchargement aisé de la carboxy-2-anthraquinone.

Au cours de la réaction, l'acide nitrique est de préférence introduit sous forme de solution concentrée, de 58 à 60 % en poids, dans le réacteur. Ceci permet de maintenir un titre de 3 à 15 % en poids en acide nitrique dans le milieu réactionnel.

La durée de la réaction dépend de la température à laquelle est effectuée la réaction. Lorsque la réaction est effectuée à 190°C dans les conditions énoncées ci-dessus, la réaction dure entre 30 minutes et 1 heure.

Il est important que l'agitation soit efficace pour assurer une bonne mise en émulsion de l'éthyl-2-anthraquinone fondue (exemple : l'éthyl-2-anthraquinone fond à environ 110°C) dans la solution aqueuse d'acide nitrique et une bonne mise en suspension de la carboxy-2-anthraquinone formée qui est solide à la température de la réaction (PF ≅ 290°C).

Le réacteur peut être en acier inoxydable ou en acier émaillé.

Au cours de la réaction, un dégagement d'oxydes d'azote peut être soit éliminé, soit recyclé en acide nitrique après oxydation à l'aide d'air ou d'oxygène sous pression.

Au terme de la réaction, la carboxy-2-anthraquinone est isolée par filtration du milieu réactionnel. Le filtrat qui est une solution d'acide nitrique relativement pure peut être réutilisé après ajustement de son titre en acide nitrique par addition d'acide nitrique concentré. On réduit ainsi substantiellement la quantité d'effluents aqueux.

La carboxy-2-anthraquinone produite est très pure, à environ 99 %, et ne nécessite qu'un lavage à l'eau, de préférence tiède, jusqu'à pH neutre, signe de l'élimination de tout l'acide nitrique résiduel.

On mesure ensuite le point de fusion de la carboxy-2-anthraquinone qui doit être compris entre 287 et 297°C assurant un degré de pureté satisfaisant et de préférence entre 292 et 296°C.

### EXEMPLE 1

Nous allons maintenant décrire un exemple de procédé de synthèse de carboxy-2-anthraquinone à partir de l'éthyl-2-anthraquinone selon un mode discontinu tel que défini plus haut.

On utilise un autoclave en inox de 1000 cm3, muni d'un manchon intérieur en PTFE et équipé d'une turbine d'agitation, d'un serpentin intérieur de refroidissement à l'eau et d'un manchon chauffant extérieur électrique.

On charge à froid 600 g d'une solution aqueuse d'acide nitrique à 15 % en poids et 58,2 g d'éthyl-2-anthraquinone. On chauffe à 190°C avec une agitation de 1000 tours/mn en maintenant la pression entre 15 et 20 bars par dégazages successifs. La température est ainsi maintenue pendant 1 heure à 190°C sous une pression de 15 à 20 bars, puis on refroidit et décomprime le réacteur. Le mélange réactionnel est filtré sur un fritté en verre et lavé avec de l'eau chaude déminéralisée jusqu'à ce que le filtrat soit à un pH=6. Le précipité lavé est ensuite séché 24 heures dans une étuve ventilée à 120°C.

On obtient finalement 57,2g de carboxy-2-anthraquinone ayant un point de fusion de 293 à 294°C. Le rendement chimique de la réaction est de 92,7 % et l'analyse RMN montre que le produit a une pureté supérieure à 99 %.

### EXEMPLE 2

Nous allons maintenant décrire un exemple de procédé de synthèse de carboxy-2-anthraquinone à partir de l'éthyl-2-anthraquinone selon un mode semi-continu tel que défini plus haut.

Pour réaliser la réaction, on utilise un réacteur de 1200 cm3, équipé d'un agitateur à pales obliques à 750 tours/mn, le tout en Hastelloy C22.

On charge ensuite le réacteur à froid avec 470 g d'eau déminéralisée et 59 g d'éthyl-2-anthraquinone. On met en route le chauffage ainsi que l'agitateur. La réaction est réalisée à 180°C et la pression est régulée de façon à ne pas dépasser les 15 bars. On injecte dans le milieu réactionnel 160 g d'acide nitrique à 58 % à 180°C à l'aide d'une pompe. La pompe est réglée de manière à ce que l'injection se fasse progressivement et de façon continue durant un laps de temps d'environ 30 minutes. La réaction est ensuite menée pendant encore 30 minutes après la fin de l'injection puis on laisse le milieu réactionnel revenir à température ambiante et on décomprime le réacteur. Après filtration du milieu réactionnel, le solide récupéré est lavé à l'eau chaude déminéralisée jusqu'à neutralité des eaux de lavage puis séché à l'étuve à 120°C.

Cette réaction permet d'obtenir 60,7 g de carboxy-2-anthraquinone soit un rendement chimique de 96 %.

L'invention permet d'obtenir à moindre coût d'importantes quantités de carboxy-2-anthraquinone grâce à un procédé de synthèse mis en oeuvre facilement et utilisant des réactifs bon marché.

Le procédé de synthèse décrit ci-dessus permet également le recyclage des effluents, réduisant ainsi les coûts en matières premières et évitant les frais de retraitement des déchets.

## Revendications

1. Procédé de synthèse de carboxy-2-anthraquinone à partir d'éthyl-2-anthraquinone par oxydation nitrique **caractérisé en ce que** :
- l'oxydation nitrique est réalisée à l'aide d'une solution aqueuse d'acide nitrique à une concentration comprise, dans le milieu réactionnel, entre 1 et 20 % en poids et de préférence entre 3 et 15 % en poids,
- la proportion d'éthyl-2-anthraquinone à traiter est comprise entre 2 et 20 % en poids de solution d'acide nitrique et de préférence entre 5 et 12 % en poids,
- la température de la réaction est comprise entre 120 et 220°C et de préférence entre 160 et 200°C,
- la pression à laquelle est réalisée la réaction est comprise entre 6 et 80 bars et de préférence entre 15 et 20 bars.

2. Procédé selon la revendication 1, **caractérisé en ce que** la pression est maintenue sensiblement constante tout au long de la réaction.

3. Procédé selon l'une quelconque des revendications précédentes, **caractérisé en ce que** les vapeurs d'oxydes d'azote produites au cours de la réaction sont recyclées en acide nitrique.

## Claims

1. Process for the synthesis of 2-carboxyanthraquinone from 2-ethylanthraquinone by oxidation with nitric acid,
**characterized in that**:
- the oxidation with nitric acid is carried out using an aqueous nitric acid solution at a concentration of, in the reaction medium, between 1 and 20% by weight and preferably between 3 and 15% by weight,
- the proportion of 2-ethylanthraquinone to be treated is between 2 and 20% by weight of nitric acid solution and preferably between 5 and 12% by weight,
- the reaction temperature is between 120 and 220°C and preferably between 160 and 200°C,
- the pressure at which the reaction is carried out is between 6 and 80 bar and preferably between 15 and 20 bar.

2. Process according to Claim 1, **characterized in that** the pressure is kept substantially constant throughout the reaction.

3. Process according to either one of the preceding claims, **characterized in that** the nitrogen oxide vapours produced during the reaction are recycled as nitric acid.

## Patentansprüche

1. Verfahren zur Herstellung von 2-Carboxyanthrachinon aus 2-Ethylanthrachinon durch Salpetersäureoxidation, **dadurch gekennzeichnet, daß** man:
- die Salpetersäureoxidation mit Hilfe einer wäßrigen Lösung von Salpetersäure bei einer Konzentration zwischen 1 und 20 Gew.-% und vorzugsweise zwischen 3 und 15 Gew.-% im Reaktionsmedium durchführt,
- das zu behandelnde 2-Ethylanthrachinon in einer Menge zwischen 2 und 20 Gew.-%, bezogen auf die Salpetersäurelösung, und vorzugsweise zwischen 5 und 12 Gew.-% einsetzt,
- bei einer Reaktionstemperatur zwischen 120 und 220°C und vorzugsweise zwischen 160 und 200°C arbeitet,
- die Reaktion bei einem Druck zwischen 6 und 80 bar und vorzugsweise zwischen 15 und 20 bar durchführt.

2. Verfahren nach Anspruch 1, **dadurch gekennzeichnet, daß** man den Druck während der gesamten Reaktion weitgehend konstant hält.

3. Verfahren nach einem der vorhergehenden Ansprüche, **dadurch gekennzeichnet, daß** man die bei der Reaktion anfallenden Stickstoffoxiddämpfe als Salpetersäure zurückführt.
